Europäisches Patentamt

(19) European Patent Office

Office européen des brevets

(11) Numéro de publication : **0 299 991 B1**

## (12) FASCICULE DE BREVET EUROPEEN

(45) Date de publication du fascicule du brevet :
16.10.91 Bulletin 91/42

(51) Int. Cl.⁵ : **A61M 5/34**

(21) Numéro de dépôt : **88900365.3**

(22) Date de dépôt : **21.12.87**

(86) Numéro de dépôt international :
**PCT/EP87/00812**

(87) Numéro de publication internationale :
**WO 88/05668 11.08.88 Gazette 88/18**

(54) **DISPOSITIF DE CONNEXION POUR UN APPAREIL DE TRANSFERT D'UN LIQUIDE.**

(30) Priorité : **05.02.87 CH 413/87**

(43) Date de publication de la demande :
**25.01.89 Bulletin 89/04**

(45) Mention de la délivrance du brevet :
**16.10.91 Bulletin 91/42**

(84) Etats contractants désignés :
**AT BE CH DE FR GB IT LI LU NL SE**

(56) Documents cités :
**EP-A- 0 047 042**
**WO-A-84/04252**
**DE-A- 2 533 594**
**US-A- 2 755 801**
**US-A- 4 240 425**

(73) Titulaire : **MEDICORP HOLDING S.A.**
**11 Boulevard du Prince Henri**
**L-2014 Luxembourg (LU)**

(72) Inventeur : **MEYER, Gabriel**
**10 A, chemin des Princes**
**CH-1222 Vesenaz (CH)**
Inventeur : **HOWALD, Ernst**
**10 B, chemin des Princes**
**CH-1222 Vesenaz (CH)**

(74) Mandataire : **Nithardt, Roland**
**Cabinet Roland Nithardt Conseils en Propriété**
**Industrielle S.A. Y-Parc Scientifique et**
**Technologique Chemin de la Sallaz Case**
**postale 3347**
**CH-1400 Yverdon-les-Bains (CH)**

## Description

La présente invention concerne un dispositif de connexion pour adapter un organe d'extrémité sur un appareil de transfert d'un liquide, en particulier d'un médicament liquide, comprenant un embout tubulaire solidaire de l'appareil de transfert du liquide et un adaptateur solidaire de l'organe d'extrémité, cet embout étant pourvu d'une cavité centrale et cet adaptateur étant associé à un insert agencé pour s'engager dans cette cavité centrale.

On connaît déjà différents dispositifs de connexion d'organes d'extrémité sur des appareils de transfert de liquide. Un de ces dispositifs est décrit par le brevet US-A-4'596'561, dans lequel l'appareil de transfert est une seringue préremplie à dose unitaire et l'organe d'extrémité est une aiguille solidaire d'un capuchon adapté sur un embout porte-aiguille disposé à l'extrémité du corps de la seringue.

Un problème crucial que posent ces dispositifs est celui du volume mort, c'est-à-dire le volume du médicament qui reste dans la seringue après l'injection. Le problème est particulièrement aigu lorsque l'appareil est équipé d'un dispositif de connexion du type tronc de cône Luer normalisé ayant des dimensions extérieures avoisinant 4 mm de diamètre et présentant une conicité de 6 % sur une longueur de 7 à 10 mm. Ces dimensions extérieures sont requises pour permettre le raccordement de l'appareil à des embouts femelles normalisés associés à des éléments aussi divers que des aiguilles, des robinets à plusieurs voies, des cathéters, etc. De façon connue en soi, ce dispositif de connexion tronconique normalisé couramment appelé "dispositif d'assemblage conique Luer 6 %", comporte un conduit central pour permettre le passage du liquide. On sait par ailleurs qu'il est techniquement impossible de reproduire rationnellement un embout de ce type par injection d'une matière thermoplastique, lorsque le conduit central a une section relativement faible, car alors l'épaisseur des parois du tronc de cône devient proportionnellement trop importante, ce qui a pour conséquence de ralentir très fortement le cycle d'injection de la matière thermoplastique, du fait que le temps de refroidissement de cette matière doit être sensiblement allongée pour éviter les retraits.

Pour réaliser un embout du type Luer dont le canal central présente un diamètre de l'ordre de 0,4 mm, de manière à obtenir un volume mort relativement faible, l'épaisseur des parois du tronc de cône devrait être au minimum d'environ 1,8 mm. Pour une épaisseur aussi importante, la durée de la phase de refroidissement de la matière synthétique serait longue et la matière aurait tendance à former des bulles. De ce fait, il serait quasiment impossible de respecter les tolérances fixées par les normes internationales concernant les embouts mâles tronconiques du type Luer 6 %.

C'est la raison pour laquelle les seringues à jeter fabriquées par injection, et actuellement commercialisées, comportent un embout Luer traversé axialement par un conduit central dont le diamètre est de l'ordre de 3 mm, ce qui permet d'obtenir une épaisseur de paroi sensiblement plus faible qui est de l'ordre de 0,5 mm. Cet impératif technologique et économique a une conséquence fâcheuse sur l'augmentation de l'espace mort à l'intérieur du conduit axial de l'embout.

Ce problème de l'espace mort a été abordé et partiellement résolu par un dispositif décrit dans la demande de brevet européen EP-A-0 047 042. Le volume mort créé par les dimensions relativement importantes du conduit axial ménagé à l'intérieur de l'embout porte-aiguille de la seringue, est partiellement comblé par l'insertion, en fin de course, d'une pièce tronconique solidaire de l'extrémité du piston. Toutefois, ce système ne permet pas de résoudre le problème de l'assemblage standard décrit ci-dessus dit assemblage conique Luer 6 %. En effet, lorsque l'on adapte un élément tronconique femelle sur l'embout tronconique mâle Luer 6 %, il reste toujours un espace entre le fond de l'élément femelle et l'extrémité de l'embout mâle, ce qui crée un espace mort incontrôlable qui est gênant voire dangereux, bien qu'utilisé dans certains cas pour créer une chambre de visualisation permettant l'observation du passage du liquide.

Le brevet américain US-A-4 240 425 propose une construction permettant de supprimer quasi totalement le volume mort dans les seringues d'injection en prévoyant un dispositif de raccordement comportant d'une part un embout tubulaire creux et d'autre part, une embase porte-aiguille, de forme sensiblement complémentaire, qui s'adapte sur ledit embout. l'inconvénient de ce système réside dans le fait que cette embase porte-aiguille ne correspond pas aux constructions standards normalisées et empêche par conséquent, le remplacement de cette aiguille spéciale par une aiguille normalisée si, pour une raison quelconque l'aiguille spéciale ne peut pas être utilisée. En effet, si l'on met en place une aiguille normalisée comportant une embase Luer 6 % sur l'embout de cette seringue, le problème de l'espace mort évoqué ci-dessus est à nouveau posé, et à l'injection, le patient ne reçoit plus la dose de médicament prévue.

la présente invention se propose de pallier l'ensemble des inconvénients ci-dessus en réalisant un dispositif de connexion du type décrit, permettant l'utilisation d'organes d'extrémité équipés de supports conformes aux normes imposées par le principe de l'assemblage du type Luer 6 %, ainsi que la résolution du problème de l'espace mort qui est particulièrement crucial pour les médicaments injectables, et notamment ceux où le volume de substances actives est extrêmement réduit.

Dans ce but, le dispositif de connexion selon l'invention est caractérisé en ce qu'il comporte au moins une gorge longitudinale périphérique agencée pour ménager entre l'insert et la paroi intérieure de l'embout un canal de passage du liquide à transférer.

Selon une première forme de réalisation de ce dispositif, l'insert est solidaire de l'adaptateur.

Selon un second mode de réalisation de ce dispositif, l'insert est indépendant de l'adaptateur.

la cavité centrale de l'embout tubulaire comporte de préférence une paroi intérieure tronconique dont les génératrices convergent vers l'appareil de transfert.

l'embout tubulaire comporte avantageusement une paroi extérieure tronconique dont les génératrices divergent vers l'appareil de transfert. Pour permettre l'utilisation d'un adaptateur standard conforme aux normes imposées par le principe d'assemblage Luer 6 %, la conicité de la paroi extérieure tronconique de l'embout tubulaire est de préférence égale à 6 %.

Selon une utilisation particulièrement avantageuse du dispositif de connexion, l'organe d'extrémité est un trocart comportant une première partie d'extrémité tronconique, une partie intermédiaire et une seconde partie d'extrémité conique, ladite première partie d'extrémité constituant ledit insert engagé dans la cavité centrale de l'embout tubulaire, la seconde partie intermédiaire présentant un diamètre extérieur supérieur au diamètre de la cavité centrale à son extrémité évasée, et les trois parties de ce trocart étant entaillées par une gorge longitudinale périphérique définissant au moins partiellement le canal de passage du liquide à transférer.

la partie intermédiaire du trocart est agencée pour être en appui contre l'extrémité libre de l'embout tubulaire.

Selon une autre forme d'utilisation avantageuse du dispositif de connexion décrit, l'organe d'extrémité est une aiguille d'injection, et l'adaptateur comporte un capuchon en forme de cloche agencé pour s'adapter de façon étanche par-dessus l'embout tubulaire et portant ladite aiguille d'injection. l'insert, avantageusement indépendant de ce capuchon, comporte de préférence une première partie d'extrémité tronconique engagée dans la cavité centrale de l'embout tubulaire, une partie intermédiaire et une seconde partie d'extrémité engagée dans la cavité intérieure dudit capuchon en forme de cloche.

Dans ce cas, la forme de la seconde partie d'extrémité de l'insert est de préférence conçue de telle manière qu'elle n'occupe qu'une partie de l'espace libre de la cavité intérieure du capuchon en forme de cloche, de manière à ménager une chambre de contrôle agencée pour permettre de voir le passage du liquide par transparence à travers la paroi du capuchon.

Dans ce cas également, la partie intermédiaire de l'insert est avantageusement agencée pour être en appui contre l'extrémité libre de l'embout tubulaire et contre la paroi intérieure dudit capuchon en forme de cloche.

Selon une forme de réalisation préférée du dispositif, l'organe d'extrémité est une buse nasale comportant une partie d'extrémité arrondie formée à l'extrémité d'un insert logé à l'intérieur de l'embout tubulaire, et le canal de passage du liquide à transférer débouche à la base de la partie d'extrémité arrondie entre les parois de l'insert et celles de l'embout tubulaire. Ce canal de passage comporte avantageusement un tronçon d'extrémité qui débouche sous un angle compris entre 30 et 90° et de préférence au moins à proximativement égal à 60°C par rapport à la surface de l'embout tubulaire et/ou de l'insert dans la zone où débouche le canal.

Selon une première forme de réalisation avantageuse l'organe d'extrémité comporte un corps avancé à l'intérieur duquel est monté un insert définissant avec la paroi intérieure de ce corps un canal du liquide, et ce corps comporte une ailette d'arrêt qui constitue une butée disposée à une distance prédéterminée de l'ouverture du canal et agencée pour déterminer la profondeur de la zone d'application du liquide à l'intérieur des fosses nasales.

Selon une deuxième forme de réalisation avantageuse, l'organe d'extrémité est un compte-gouttes, et l'insert est pourvu d'un élément plat disposé à son extrémité supérieure, cet élément plat comportant une entaille qui prolonge le canal de passage du liquide ménagé entre ledit insert et l'embout tubulaire. Cet organe d'extrémité est de préférence surmonté d'un capuchon comportant un bourrelet intérieur agencé pour assurer, au capuchon, une position d'ouverture permettant le passage de gaz contenu à l'intérieur du distributeur et une position de fermeture étanche. Ledit capuchon peut comporter un fond associé à une substance bactéricide.

La présente invention sera mieux comprise en référence à la description d'exemples de réalisation et du dessin annexé dans lequel :

la figure 1 représente une première forme de réalisation du dispositif selon l'invention où l'organe d'extrémité est un trocart,

la figure 1A est une vue en coupe transversale selon la ligne I-I de la fig. 1,

la figure 1B représente une vue en coupe transversale selon la ligne II-II de la figure 1,

la figure 1C représente une vue en coupe transversale selon la ligne III-III de la figure 1,

la figure 2 représente une seconde forme de réalisation du dispositif selon l'invention où l'organe d'extrémité est une aiguille d'injection d'un médicament liquide,

la figure 2A représente une vue en coupe transversale selon la ligne IV-IV de la figure 2,

la figure 2B est une vue en coupe transversale selon la ligne V-V de la figure 2,

la figure 2C est une vue en coupe transversale selon la ligne VI-VI de la figure 2,

la figure 3 représente une variante du dispositif tel que représenté par la figure 1,

la figure 4 représente une variante du dispositif tel que représenté par la figure 2,

la figure 5 représente un autre dispositif de connexion selon l'invention, utilisable comme buse nasale,

la figure 6 représente une variante du dispositif de connexion selon l'invention, également utilisable comme buse nasale,

la figure 7 représente une autre forme de réalisation du dispositif de connexion selon l'invention, utilisable comme compte-gouttes, et

les figures 8 et 9 représentent un capuchon de fermeture du compte-gouttes de la figure 7, respectivement en position d'ouverture et de fermeture.

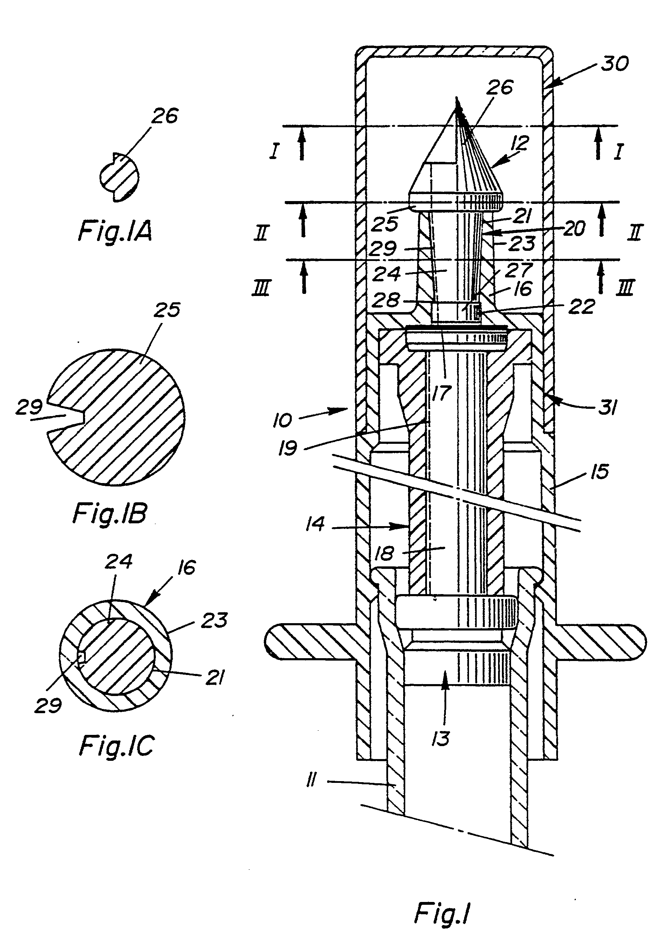

La figure 1 représente un appareil 10 de transfert d'un liquide comportant essentiellement un récipient 11 contenant par exemple un liquide destiné à être transféré dans un autre récipient contenant une autre substance liquide ou solide, telle que par exemple un lyophilisat. Cette autre substance peut être contenue dans un flacon ou sachet obturé par une membrane destinée à être percée par un trocart 12 qui constitue l'organe d'extrémité associé à l'appareil de transfert. Cet appareil de transfert comporte en outre un piston 13 monté à l'extrémité d'une tige 14 fixée, par exemple par soudure aux ultrasons ou tout autre moyen approprié, au fond d'une capsule 15 pourvue à son extrémité libre d'un embout tubulaire 16 dans lequel est logé ledit trocart 12. Un filtre à membrane 17 est monté entre le fond de la capsule 15 et la tige 14. Cette dernière est creuse et contient un noyau 18, sensiblement cylindrique pourvu d'une gorge axiale périphérique 19 qui forme avec la paroi intérieure de la tige 14 un canal longitudinal permettant l'écoulement du liquide à travers ladite tige en direction du filtre 17.

L'embout tubulaire 16 comporte une cavité centrale 20 qui est définie par une paroi intérieure 21 de forme tronconique prolongée à son extrémité adjacente au fond de la capsule 15 par une paroi annulaire cylindrique 22. La paroi tronconique 21 a des génératrices convergeant en direction de l'appareil de transfert. La paroi extérieure 23 de l'embout tubulaire 16 est également tronconique, mais ses génératrices sont divergentes en direction de l'appareil de transfert. La paroi extérieure 23 présente de préférence une conicité de 6 % pour être adaptée aux normes internationales de l'assemblage du type Luer 6 %.

Le trocart 12 comporte au moins une première partie tronconique 24 destinée à être logée à l'intérieur de l'embout tubulaire 16. Cette partie comporte une surface latérale tronconique dont la conicité est égale à celle de la paroi intérieure 21 de l'embout tubulaire 16. Il comporte par ailleurs une partie intermediaire 25, sensiblement cylindrique, dont le diamètre est supérieur au diamètre intérieur de la cavité centrale de l'embout tubulaire 16, dans sa zone la plus évasée. Bien que le diamètre de la partie intermédiaire 25 pourrait être égal, voire inférieur au diamètre extérieur de l'embout tubulaire 16, l'avantage du diamètre supérieur est qu'il permet de supprimer le risque d'une adaptation erronee d'une aiguille standard sur un appareil de transfert contenant une substance devant être diluée avant injection. Le trocart 12 comporte enfin une troisième partie 26 conique destinee à permettre le percement d'une membrane ou d'un couvercle d'un récipient prévu pour recevoir le liquide à transférer, initialement contenu dans le récipient 11.

Dans l'exemple illustré, la partie tronconique 24 du trocart est prolongee par une bague cylindrique 27 définissant un épaulement annulaire 28 avec la partie 24 et destinee à s'engager dans l'évidement cylindrique limite par la paroi cylindrique 22 et ménage à la base de l'embout tubulaire 16 et à travers le fond de la capsule 15.

Dans cet exemple de réalisation, l'organe d'extrémité est un trocart dont le rôle a été défini ci-dessus. L'adaptateur solidaire de l'organe d'extrémité est en quelque sorte constitué par la pointe ou partie conique 26 de ce trocart. l'adaptateur est confondu avec l'insert mentionné précédemment et ces deux éléments sont réunis en un seul élement qui est la partie tronconique 24 du trocart prolongee par la bague cylindrique 27.

Pour permettre au liquide ayant traversé le filtre 17 de s'écouler en direction de la pointe du trocart 12, la bague cylindrique 27, l'élément tronconique 24, l'élément intermédiaire 25 et l'élément conique 26 du trocart comportent une gorge longitudinale périphérique 29 qui coopere avec les parois 22 et 21 respectivement ménagées dans le fond de la capsule 15 et à l'intérieur de l'embout tubulaire 16 pour former un canal de très faible section, permettant l'écoulement du liquide tout en évitant de former un volume mort important pour l'appareil de transfert concerné.

Un capuchon de protection 30 peut être adapté par-dessus le trocart 12 et fixe par des moyens connus en soi à la capsule 15 qui comporte un evidement 31 approprie à sa base.

La bague cylindrique 27 constitue un organe de retenue qui coopere avec l'épaulement 28 pour assurer le maintien en position du trocart 12 et le serrage de la partie intermediaire 25 contre l'extrémité libre de l'embout tubulaire 16. De ce fait, l'étanchéite a ce niveau est parfaitement assuree.

Le même principe de construction est utilise pour creer le conduit longitudinal 19, ce qui permet de réduire au strict minimum le volume mort de l'appareil de transfert, c'est-a dire le volume de liquide residuel après l'opération de transfert.

Les figures 1A, 1B et 1C illustrent au moyen des vues en coupe, respectivement selon les lignes I-I, II-II et III-III, la geométrie des différentes parties du trocart

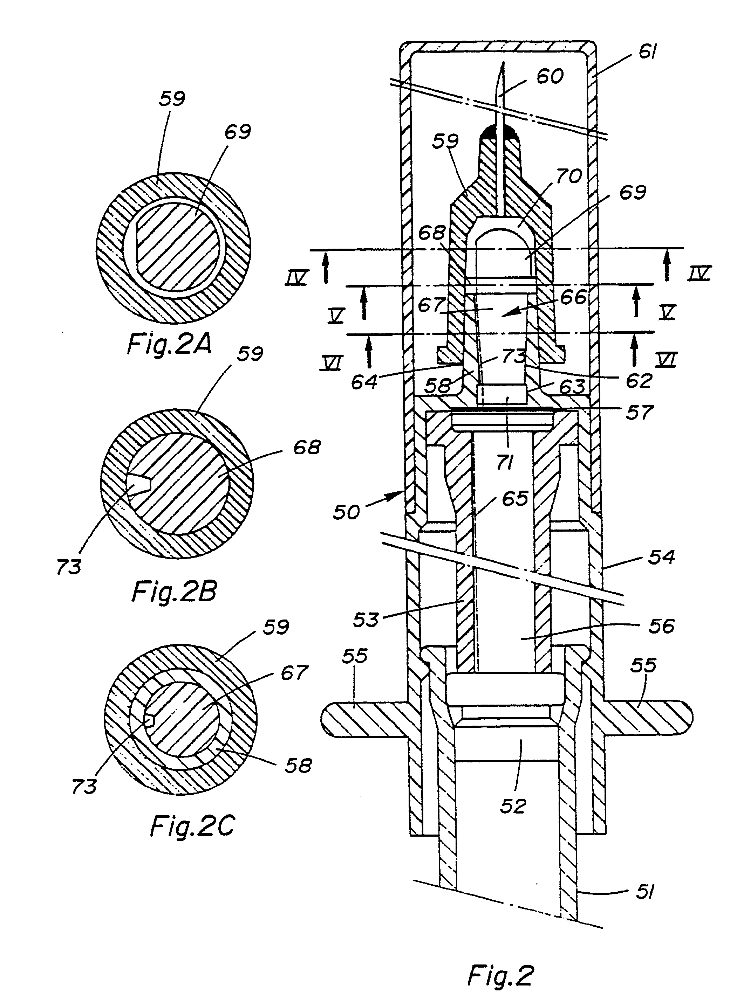

La fig. 2 represente un appareil 50 de transfert d'un liquide, se presentant sous la forme d'une seringue du type jetable a dose unitaire ou multiple. Cette seringue comporte essentiellement un récipient 51 contenant le medicament injectable, un piston 52 engage a l'extremite du récipient 51 et monté sur une tige 53 de forme sensiblement cylindrique fixee, par exemple par soudure aux ultrasons, au fond d'une capsule 54 comportant deux ailettes 55 de préhension de la seringue La tige 53 est creuse et comporte un noyau 56 de forme sensiblement cylindrique. Un filtre 57 est positionné au fond de la capsule 54. Cette capsule se prolonge par un embout tubulaire 58 qui constitue un embout porte-aiguille agence pour recevoir un capuchon 59 en forme de cloche qui porte une aiguille 60. Avant son utilisation, un capuchon de protection est monte a l'extremite de la capsule 54 pour maintenir l'aiguille dans un milieu stérile

L'embout tubulaire 58 a la même forme et les mêmes dimensions que l'embout tubulaire 16 decrit en reference a la fig. 1. Sa surface inférieure 62 est sensiblement tronconique, et prolongee a son extremite inférieure par une paroi annulaire cylindrique 63. Sa surface extérieure 64 est egalement tronconique et présente une conicité de 6% permettant le raccordement d'un élement porte-aiguille standard, dans le but d'autoriser un assemblage du type Luer a 6%. Le capuchon 59 presente une conicite intérieure de 6% et s'adapte parfaitement sur l'embout tubulaire 58.

Le noyau 56, loge a l'interieur de la tige tubulaire 53, permet d'éviter un volume mort trop important le médicament qui passe a travers le piston 52 au moyen d'un conduit (non représente), s'ecoule axialement à travers un canal longitudinal défini par une gorge ménagée a la peripherie du noyau 56 et par la paroi interieure de la tige cylindrique creuse 53. Dans ce même but, c'est à dire pour limiter le volume mort, à savoir le volume de medicament restant dans la seringue apres l'injection, un insert 66 est engage dans la cavité tronconique definie par l'embout tubulaire 58. Cet insert se compose essentiellement d'une première partie tronconique 67 entierement engagee a l'intérieur de l'embout tubulaire 58, d'une partie intermediaire 68 qui s'appuie, d'une part sur la surface annulaire d'extrémité de l'embout tubulaire 58 et, d'autre part sur la paroi intérieure du capuchon 59 portant l'aiguille 60, pour former une sorte de joint d'étanchéité entre ces deux composants l'insert comporte une troisieme partie 69 dont la forme et les dimensions sont étudiées de maniere a ne pas remplir en totalité le volume de la partie restante de la cavité intérieure du capuchon 59, de manière a former une chambre de visualisation permettant de voir le médicament a injecter au moment de l'injection, à travers la paroi transparente ou semi transparente du capuchon 59. L'extremité inférieure de la première partie tronconiqie 67 de l'insert 66 comporte un élément cylindrique 71 dont le but est de former un organe de retenue maintenant l'insert en position dans l'embout tubulaire 58. Une gorge 73 est ménagee a la peripherie de l'insert 66 pour permettre l'écoulement du liquide a injecter ayant traverse le filtre 57 et pour l'amener dans la chambre de visualisation qui communique avec l'extrémite inferieure de l'aiguille 60.

Les fig. 2A, 2B et 2C, qui representent des vues en coupe selon les lignes IV-IV, V-V et VI-VI, illustrent respectivement la forme du capuchon 59, de la partie superieure 69 de l'insert 66, de la partie intermédiaire 68 de cet insert, de la partie inférieure 67 de l'insert 66 et enfin du canal 73.

Dans ce cas, l'insert 66 est une pièce independante engagee de force a l'intérieur de l'embout tubulaire 58 et destinée a limiter au minimum le volume mort, c'est-a dire le volume résiduel de médicament injectable apres utilisation de la seringue, tout en permettant l'utilisation d'aiguilles standards avec assemblage du type Luer 6%.

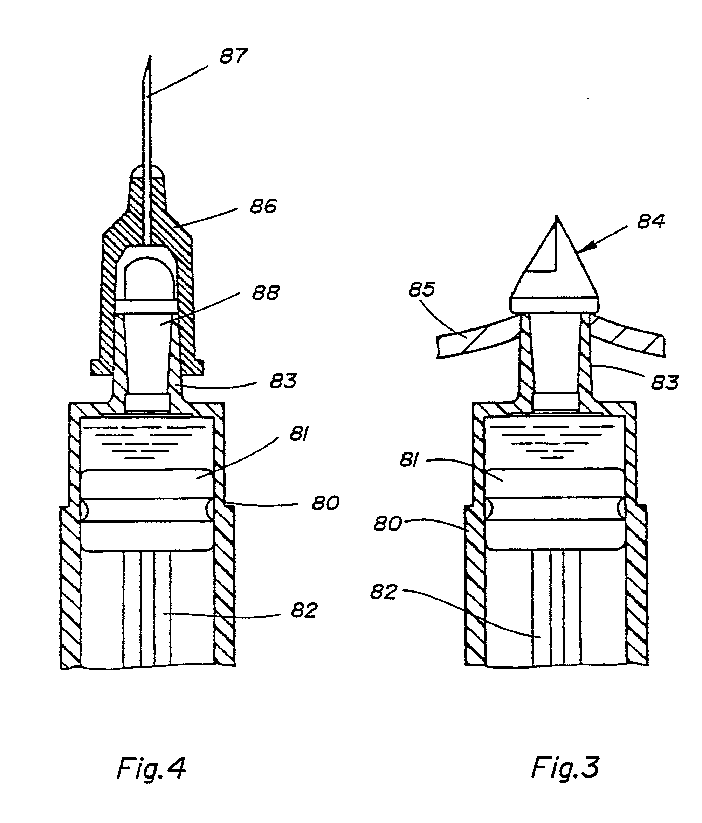

La fig. 3 représente une variante du dispositif represente par la fig. 1. Une seringue conventionnelle 80 a piston 81, fixee a l'extremité d'une tige de piston 82, est equipée d'un embout tubulaire 83 identique a celui décrit en reference a la fig. 1. Cet embout reçoit un trocart 84 qui est en tout point identique a celui décrit precédemment. Sur cette figure, le trocart 84 a ete utilisé pour perforer une membrane 85.

La fig. 4 represente une seringue 80, identique a celle de la fig. 3, comportant un piston 81 fixe à l'extrémité d'une tige de piston 82 et equipée d'un embout tubulaire 83. Sur cet embout est fixé un capuchon 86 portant une aiguille 87, qui sont strictement identiques respectivement au capuchon 59 et a l'aiguille 60 représentes par la fig. 2. Un insert 88 est loge a l'intérieur de l'embout d'extremité 83. Les dispositifs des fig. 3 et 4 permettent de demontrer que l'embout tronconique tel que décrit ci-dessus, destine a permettre un assemblage standard du type Luer 6%, peut être adapte sur n'importe quel appareil de transfert connu.

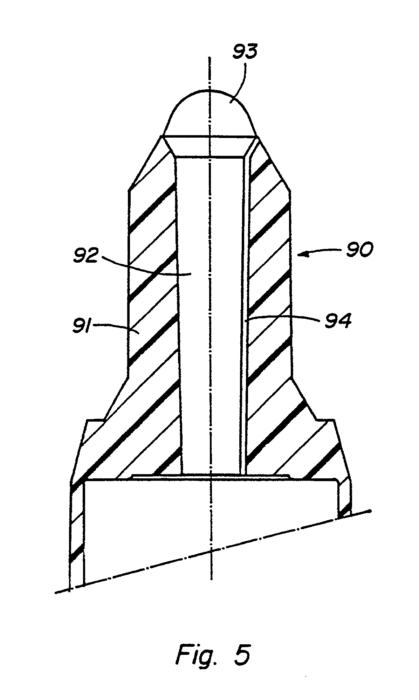

Dans l'exemple represente par la fig. 5, le dispositif de connexion 90 est destine a être utilise comme buse nasale, c'est-à-dire comme distributeur d'un médicament sur les muqueuses nasales Il comporte comme precédemment un embout tubulaire 91 pourvu d'une ouverture axiale centrale dans laquelle est engage un insert 92. L'ouverture centrale de l'embout tubulaire 91 est légèrement conique. Il en est de même de la partie inferieure de l'insert destinee a être engagee dans cette ouverture L'extrémite superieure de l'insert 92 présente une forme arrondie sensiblement sphérique qui peut entrer en contact avec les muqueuses sans risquer de les blesser. Un conduit 94 est menagé entre la paroi inferieure de l'embout tubulaire 91 et la paroi extérieure de l'insert 92, pour assurer l'ecoulement du liquide médicamenteux initialement contenu dans le recipient (non représente) sur lequel est adapte le dispositif de connexion 90. Ce canal sensiblement parallèle a l'axe de l'insert dans sa partie inférieure dévie et forme un angle d'environ 60° au voisinage de son extremité superieure. Cette inclinaison permet de deposer le médicament directement sur les muqueuses nasales et d'assurer ainsi une bonne absorption du produit actif.

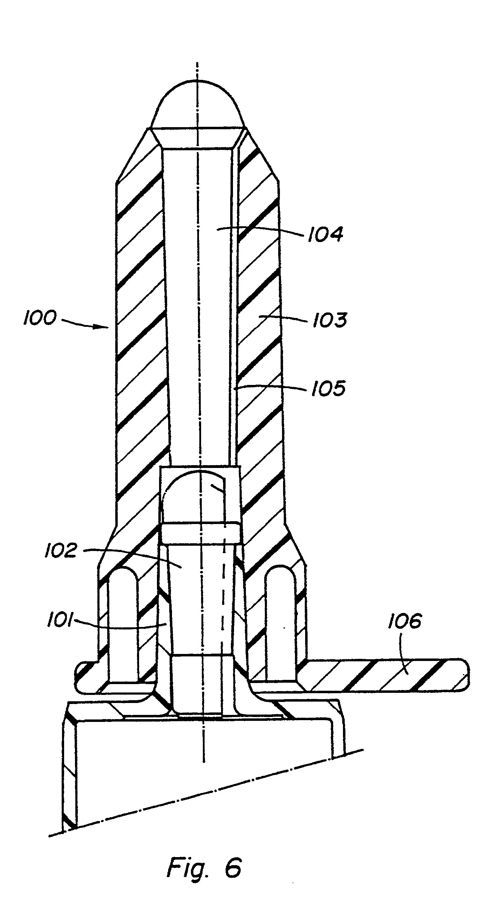

La fig. 6 illustre un autre mode de realisation d'une buse nasale, qui est en quelque sorte une combinaison du dispositif illustré par la fig. 2 avec celui de la fig. 5. En effet, ce dispositif de connexion 100 comporte un embout tubulaire 101 a l'intérieur duquel est engage un insert 102 qui est sensiblement identique a l'insert 66 du dispositif de la fig. 2. La buse nasale proprement dite se compose d'un corps 103 qui s'emboîte sur l'embout tubulaire 101, par-dessus l'insert 102. Ce corps tubulaire comporte un alésage central qui est obture en grande partie par un deuxième insert 104, sensiblement identique a l'insert 92 représenté par la fig. 5, et qui ménage, entre la paroi interieure du corps 103 et la paroi extérieure dudit deuxième insert 104, un canal latéral 105 dont la forme et la fonction sont sensiblement identiques a celles du canal 94 du dispositif de la figure précedente. A sa base, le corps 103 est pourvu d'une ailette 106 qui constitue une butee dont le rôle est de definir la profondeur a laquelle est enfoncee la buse nasale dans le nez, c'est-à-dire la zone dans laquelle sera distribue le médicament. Cette profondeur est choisie de telle manière que la zone de distribution du médicament corresponde à une zone preférentielle d'absorption des muqueuses nasales.

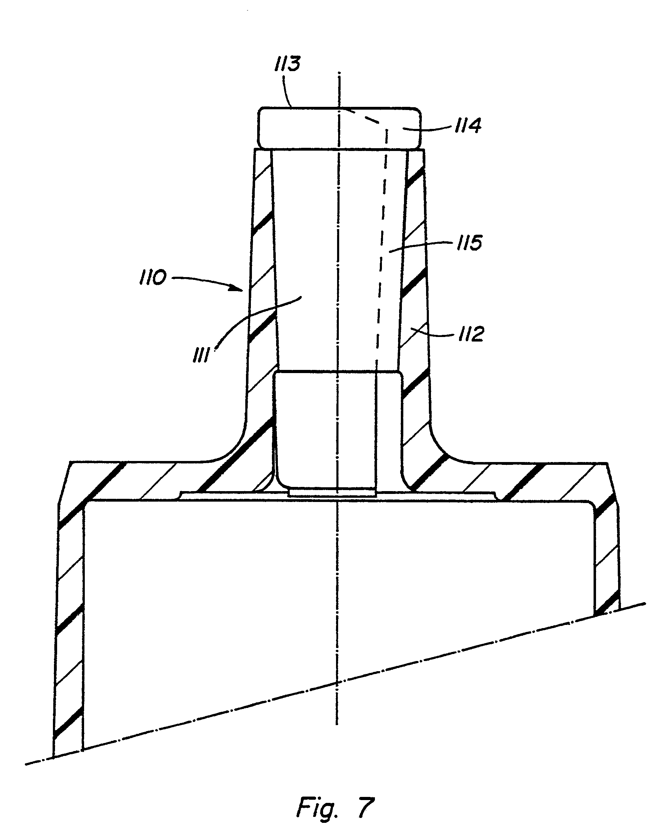

La fig. 7 illustre un autre dispositif de connexion 110 qui a une fonction de compte-gouttes. Dans cet exemple, l'insert 111, qui est loge a l'intérieur de l'embout tubulaire 112, est surmonté d'un élement plat 113 pourvu d'une entaille periphérique 114 qui communique avec le canal longitudinal 115 limité par la paroi intérieure de l'embout tubulaire 112 et par la paroi d'une entaille axiale menagee dans l'insert 111.

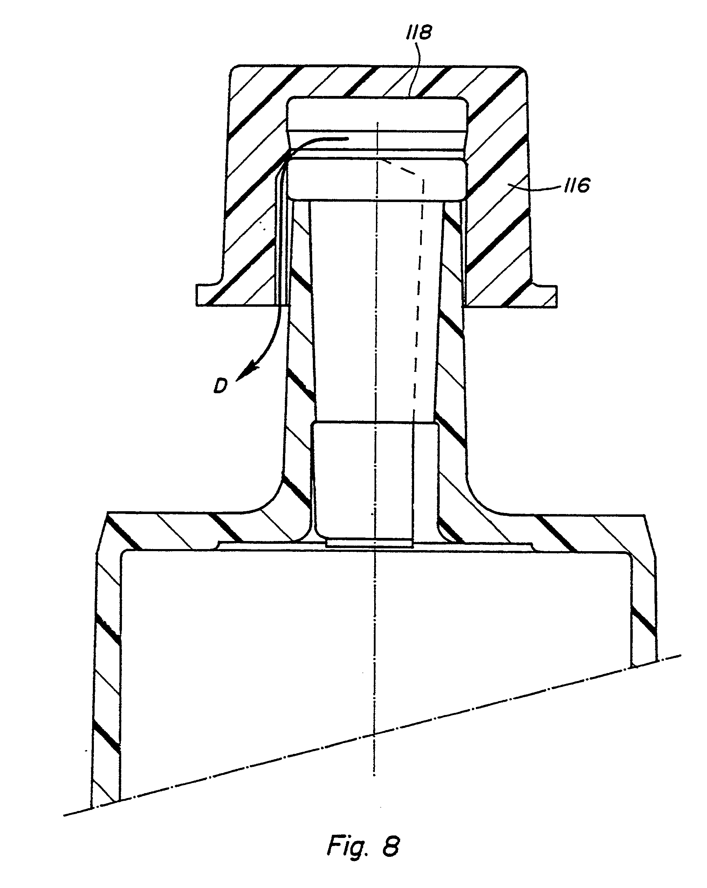

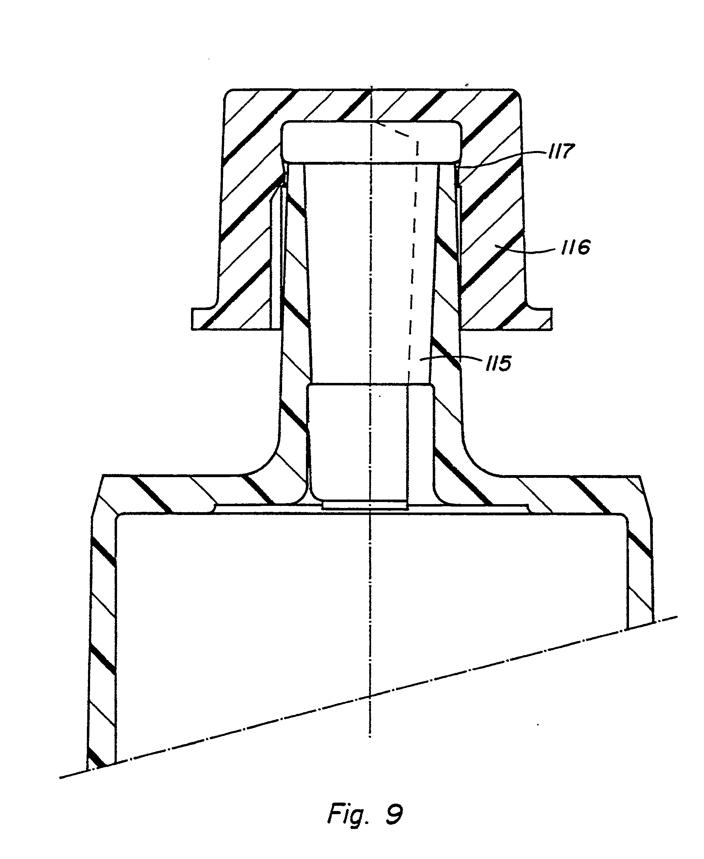

Les fig. 8 et 9 reprennent le dispositif representé par la fig. 7 et lui associent un capuchon de fermeture étanche 116. Sur la fig. 8, ce capuchon 116 occupe une position ouverte permettant le degazage, c'est-à dire l'evacuation de l'air contenu à l'intérieur du corps du distributeur dans le sens de la fleche D. Sur la fig. 9, le capuchon 116 est represente dans sa position de fermeture dans laquelle il assure une obturation étanche au canal distributeur 115. A cet effet, la paroi latérale intérieure du capuchon 116 comporte une protubérance annulaire 117 qui peut être amenée, grâce a l'élasticite relative de la matière, par-dessus l'élement plat 114 qui surmonte l'insert 11 pour prendre appui, en dessous de cet élement plat, contre la paroi extérieure de l'embout tubulaire 112. Le fond 118 de la cavité intérieure du capuchon 116 peut être traité au moyen d'une substance bactericide, ou peut porter une plaquette d'une substance bactéricide qui ssure une désinfection permanente de la zone d'écoulement du compte-gouttes. Cet agencement est particulièrement avantageux dans un système compte-gouttes qui est destine à être utilise à de nombreuses reprises, chaque utilisation amenant le compte-gouttes en contact avec l'environnement, et permettant par consequent une contamination Entre les différentes utilisations, la periode de stockage permet l'action de la susbtance bactéricide et engendre de ce fait une décontamination systématique de la zone contaminee.

Il est bien entendu que la présente invention n'est pas limitée aux formes de realisation décrites, mais peut subir différentes modifications et se présenter sous diverses variantes évidentes pour l'homme de l'art. C'est ainsi que la forme et les dimensions des différents composants du compte-gouttes ou des buses nasales, ainsi que du capuchon protecteur et eventuellement bactéricide, peuvent être modifiées en fonction des besoins ou en fonction des impératifs de fabrication.

Dans le cas de la buse nasale, les parametres, butée, inclinaison de la zone d'extrémité du canal et diamètre de ce canal, peuvent être modifiés en fonction du type de medicament administre et de sa viscosite. La position de l'ailette de butée illustree par la fig. 6 depend egalement de la position de la zone preférentielle d'absorption des muqueuses nasales. Dans le cas du compte gouttes, la forme extérieure de l'insert peut être adaptée a l'utilisation prevue pour ce compte-gouttes. Le but est de déposer une goutte calibree, dans les yeux, a la surface du corps, dans la bouche ou encore dans un verre en vue de sa dilution dans de l'eau ou dans un autre liquide a absorber. l'avantage fondamental d'un tel compte gouttes, monté sur un distributeur à piston, est que l'evacuation de la goutte est commandee par l'action du piston. Contrairement aux compte gouttes classiques, il n'y a pas reabsorption de gaz a l'interieur du corps du distributeur et par consequent pas de risque de contamination de la solution medicamenteuse contenue a l'intérieur de ce distributeur.

## Revendications

1. Dispositif de connexion pour adapter un organe d'extrémité sur un appareil (10,50) de transfert d'un liquide, en particulier d'un médicament liquide, comprenant un embout tubulaire ( 16, 58, 83, 91, 101, 112) solidaire de l'appareil ( 10, 50) de transfert du liquide et un adaptateur solidaire de l'organe d'extrémité (12, 60, 87, 84, 93, 113), cet embout étant pourvu d'une cavité centrale et cet adaptateur étant associé à un insert (24, 66, 88, 92, 104, 111) agencé pour s'engager dans cette cavité centrale, caractérisé en ce qu'il comporte au moins une gorge longitudinale périphérique (29, 73, 94, 105, 115) agencée pour ménager entre l'insert et la paroi intérieure de l'embout un canal de passage du liquide à transférer.

2. Dispositif selon la revendication 1, caractérisé en ce que l'insert (24) est solidaire de l'adaptateur.

3. Dispositif selon la revendication 1, caractérisé en ce que l'insert (66) est indépendant de l'adaptateur.

4. Dispositif selon la revendication 1, caractérisé en ce que la cavité centrale de l'embout tubulaire (16, 58) comporte une paroi intérieure tronconique (21, 62) dont les génératrices convergent vers l'appareil de transfert (10, 50).

5. Dispositif selon la revendication 1, caractérisé en ce que l'embout tubulaire (16, 58) comporte une paroi extérieure tronconique (23, 64) dont les génératrices divergent vers l'appareil de transfert.

6. Dispositif selon la revendication 5, caractérisé en ce que la conicité de la paroi extérieure tronconique (23, 64) de l'embout tubulaire (16, 58) est de 6%.

7. Dispositif selon la revendication 1, caractérisé en ce que l'organe d'extrémité est un trocart (12), comportant une première partie d'extrémité tronconique (24), une partie intermédiaire (25) et une seconde partie d'extrémité conique (26), en ce que cette première partie d'extrémité (24) constitue ledit insert et s'engage dans la cavité centrale (20) de l'embout tubulaire (16), en ce que cette partie intermédiaire (25) présente un diamètre supérieur au diamètre de la cavité centrale, à son extrémité evasée, et en ce que les trois parties de ce trocart sont entaillees par une gorge longitudinale peripherique (29) definissant au moins partiellement le canal de passage du liquide a transferer.

8. Dispositif selon la revendication 6, caractérise en ce que la partie intermediaire (25) du trocart est agencee pour être en appui contre l'extremite libre de l'embout tubulaire ( 16).

9. Dispositif selon la revendication 1, caracterisé en ce que l'organe d'extrémité est une aiguille d'injection (60), en ce que l'adaptateur est un capuchon (59) en forme de cloche agence pour s'adapter de façon etanche par-dessus l'embout tubulaire (58) et portant cette aiguille d'injection et en ce que l'insert (66), indépendant de ce capuchon comporte une premiere partie d'extrémite tronconique (67) engagee dans la cavite centrale de l'embout tubulaire (58), une partie intermédiaire (68) et une seconde partie d'extremite (69) engagee dans la cavite intérieure dudit capuchon en forme de cloche.

10. Dispositif selon la revendication 9, caracterise en ce que la forme de la seconde partie d'extremite (69) de l'insert (66) est conque de telle maniere qu'elle n'occupe qu'une partie de l'espace libre de la cavite interieure du capuchon (59) en forme de cloche, de maniere a menager une chambre de contrôle agencee pour permettre de voir le passage du liquide par transparence a travers la paroi du capuchon.

11. Dispositif selon la revendication 9, caracterise en ce que la partie intermedaire (68) de l'insert (66) est agencee pour être en appui contre l'extrémité libre de l'embout tubulaire (58) et contre la paroi interieure dudit capuchon en forme de cloche.

12. Dispositif selon la revendication 1, caracterise en ce que. l'organe d'extremite est une buse nasale (90) comportant une partie d'extremite arron-die (93) formée à l'extremite d'un insert (92) loge à l'intérieur de l'embout tubulaire (91), et en ce que le canal de passage du liquide a transférer debouche a la base de la partie d'extremite arrondie (93) entre les parois de l'insert (92) et celles de l'embout tubulaire (91).

13. Disposititif selon la revendication 12, caracterise en ce que le canal de passage (94) comporte un tronçon d'extremite qui debouche sous un angle compris entre 30 et 90° et de preference au moins approximativement egal a 60°C par rapport a la surface de l'embout tubulaire et/ou de l'insert dans la zone ou debouche le canal.

14. Dispositif selon la revendication 12, caracterise en ce que l'organe d'extrémite comporte un corps avance (103) a l'interieur duquel est monte un insert (104) définissant avec la paroi interieure de ce corps un canal (105) du liquide, et en ce que ce corps comporte une ailette d'arrêt (106) qui constitue une butee disposee a une distance predéterminee de l'ouverture du canal (105) et agencee pour determiner la profondeur de la zone d'application du liquide à l'interieur des fosses nasales.

15. Dispositif selon la revendication 1, caracterisé en ce que l'organe d'extremité est un compte-gouttes (110), et en ce que l'insert (111) est pourvu d'un elément plat (113) dispose a son extremitè supérieure, cet elément plat comportant une entaille (114) qui prolonge le canal de passage du liquide (115) ménage entre ledit insert et l'embout tubulaire (112).

16. Dispositif selon la revendication 15, caracterisé en ce que l'organe d'extrémité est surmonté d'un capuchon (116) comportant un bourrelet intérieur ( 117) agence pour assurer, au capuchon, une position d'ouverture permettant le passage de gaz contenus a l'intérieur du distributeur et une position de fermeture étanche.

17. Dispositif selon la revendication 16, caracterisé en ce que le capuchon (116) comporte un fond associe à une substance bactericide.

**Patentansprüche**

1. Verbindung für die Anpassung eines Endstücks auf eine Übertragungsvorrichtung (10, 50) für Flüssigkeit, insbesondere für eine flüssige Arznei, bestehend aus einem rohrförmigen Ansatzstück (16, 58, 83, 91, 101, 112), das mit der Übertragungsvorrichtung (10, 50) für Flüssigkeit fest verbunden ist, und einem Adapter, der mit dem Endstück (12, 60, 87, 84, 93, 113) fest verbunden ist, wobei das besagte Ansatzstück mit einem zentralen Hohlraum versehen und der besagte Adapter mit einem Einsatzstück (24, 66, 88, 92, 104, 111) verbunden ist, das eingerichtet ist, um in diesen zentralen Hohlraum eingeführt zu werden, **dadurch gekennzeichnet**, daß sie mindestens eine peripherische Längsnut (29, 73, 94, 105, 115) enthält, die eingerichtet ist, um zwischen dem Einsatzstück und der inneren Wandung des Ansatzstücks einen Kanal für das Abfließen der Flüssigkeit zu bilden, die zu übertragen ist.

2. Verbindung gemäß Anspruch 1, **dadurch gekennzeichnet**, daß das Einsatzstück (24) mit dem Adapter fest verbunden ist.

3. Verbindung gemäß Anspruch 1, **dadurch gekennzeichnet**, daß das Einsatzstück (66) von dem Adapter unabhängig ist.

4. Verbindung gemäß Anspruch 1, **dadurch gekennzeichnet**, daß der zentrale Hohlraum des rohrförmigen Ansatzstückes (16, 58) eine kegelstumpfförmige innere Wandung (21, 62) enthält, deren Erzeugende zu der Übertragungsvorrichtung (10, 50) zusammenlaufen.

5. Verbindung gemäß Anspruch 1, **dadurch gekennzeichnet**, daß das rohrförmige Ansatzstück (16, 58) eine kegelstumpfförmige äußere Wandung (23, 64) enthält, deren Ergzeugende zu der Übertragungsvorrichtung auseinanderlaufen.

6. Verbindung gemäß Anspruch 5, **dadurch gekennzeichnet**, daß die Konizität der kegelstumpfförmigen äußeren Wandung (23, 64) des rohrförmigen Ansatzstücks (16, 58) 6 % beträgt.

7. Verbindung gemäß Anspruch 1, **dadurch gekennzeichnet**, daß das Endstück ein Trokar (12) mit einem ersten kegelstumpfförmigen Endteil (24), einem Zwischenteil (25) und einem zweiten kegelstumpfförmigen Endteil (26) ist, daß der besagte erste Endteil (24) das besagte Einsatzstück bildet und in den zentralen Hohlraum (20) des rohrförmigen Ansatzstücks (16) eingeführt wird, daß der besagte Zwischenteil (25) einen Durchmesser aufweist, der größer als der Durchmesser des zentralen Hohlraums ist und an dessen Ende auslaufend ist, und daß die drei Teile dieses Trokars durch eine peripherische Längsnut (29) angeschnitten sind, die den Kanal für das Abfließen der Flüssigkeit mindestens teilweise festlegt, die zu übertragen ist.

8. Verbindung gemäß Anspruch 6, **dadurch gekennzeichnet**, daß der Zwischenteil (25) des Trokars eingerichtet ist, um gegen das freie Ende des rohrförmigen Ansatzstücks (16) aufzuliegen.

9. Verbindung gemäß Anspruch 1, **dadurch gekennzeichnet**, daß das Endstück eine Spritznadel (60) ist, daß der Adapter eine glockenförmige Kappe (59) ist, der eingerichtet ist, um über dem rohrförmigen Ansatzstück (58) dicht aufgesetzt zu werden, und die besagte Spritznadel (60) trägt, und daß das Einsatzstück (66), unabhängig von dieser Kappe, einen ersten kegelstumpfförmigen Teil (67), der in dem zentralen Hohlraum des rohrförmigen Ansatzstücks (58) eingeführt ist, einen Zwischenteil (68) und einen zweiten kegelstumpfförmigen Endteil (69), der in dem inneren Hohraum der besagten glockenförmigen Kappe eingeführt ist, enthält.

10. Verbindung gemäß Anspruch 9, **dadurch gekennzeichnet**, daß die Form des zweiten Endteils (69) des Einsatzstücks (66) derart ausgelegt ist, daß er nur einen Teil des freien Raums des inneren Hohlraums der glockenförmigen Kappe (59) ausfüllt, um eine Kammer zur Kontrolle zu bilden, die eingerichtet ist, um durch Transparenz das Abfließen der Flüssigkeit durch die Wandung der Kappe zu sehen.

11. Verbindung gemäß Anspruch 9, **dadurch gekennzeichnet**, daß der Zwischenteil (68) des Einsatzstücks (66) eingerichtet ist, um gegen das freie Ende des rohrförmigen Ansatzstücks (58) und gegen die innere Wandung der besagten glockenförmigen Kappe aufzuliegen.

12. Verbindung gemäß Anspruch 1, **dadurch gekennzeichnet**, daß das Endstück ein Rohr für die Nase (9) ist mit einem abgerundeten Endteil (93), der an dem Ende eines Einsatzgitters (92) gebildet ist, das innerhalb des rohrförmigen Ansatzstücks (91) angeordnet ist, und daß die Nut (Kanal) für das Abfließen der Flüssigkeit, die zu übertragen ist, an der Basis des abgerundeten Endteils (93) zwischen den Wandungen des Einsatzstücks (92) und den Wandungen des rohrförmigen Ansatzstücks mündet.

13. Verbindung gemäß Anspruch 12, **dadurch gekennzeichnet**, daß die Nut (Kanal) für das Abfließen (94) einen Endstrang (Endstück) enthält, der unter einem Winkel zwischen 30° und 90° und vorzugsweise mindestens annähernd von 60° im Verhältnis zu der Fläche des rohrförmigen Ansatzstücks und/oder des Einsatzstücks in den Bereich, in den der Kanal ausläuft, mündet.

14. Verbindung gemäß Anspruch 12, **dadurch gekennzeichnet**, daß das Endstück einen vorgeschobenen Körper (103) enthält, in dem ein Einsatzstück (104) angeordnet ist, das mit der inneren Wandung dieses Körpers einen Kanal (105) für die Flüssigkeit bildet, und daß dieser Körper (103) einen Sperrflügel (106) enthält, der als Anschlag dient, der bei einem vorher bestimmten Abstand von der Öffnung des Kanals (105) angeordnet und eingerichtet ist, um die Tiefe des Anwendungsbereichs der Flüssigkeit innerhalb der Nasenhöhlen zu bestimmen.

15. Verbindung gemäß Anspruch 1, **dadurch gekennzeichnet**, daß das Endstück ein Tropfenzähler (110) ist, und daß das Einsatzstück (111) mit einem flachen Teil (113) versehen ist, der an seinem oberen Ende angeordnet ist, wobei dieser flache Teil eine Kerbe (114) enthält, die den Kanal für das Abfließen der Flüssigkeit (115), der zwischen dem besagten Einsatzstück und dem rohrförmigen Ansatzstück (112) eingerichtet ist, verlängert.

16. Verbindung gemäß Anspruch 15, **dadurch gekennzeichnet**, daß das Endstück eine Kappe (116) trägt, die eine innere Wulst (117) enthält, die eingerichtet ist, damit die Kappe eine geöffnete Stellung für das Durchströmen von Gasen, die innerhalb des Verteilers enthalten sind, und eine abgedichtete geschlossene Stellung einnimmt.

17. Verbindung gemäß Anspruch 16, **dadurch gekennzeichnet**, daß die Kappe einen Boden, der mit einem bakterientötenden Stoff verbunden ist, enthält.

## Claims

1. Connecting device for adapting a tip to a liquid transfer device (10, 50), particularly liquid medication, comprising a tubular ferrule (16, 58, 83, 91, 101, 112) forming a unit with the transfer device (10, 50) and an adapter forming a unit with the tip (12, 60, 84, 87, 93, 113), said ferrule being provided with a central cavity and said adapter being associated with an insert (24, 66, 88, 92, 104, 111) disposed to engage in said central cavity, characterized in that it comprises at least one longitudinal, peripheral groove (29, 73, 94, 105, 115) disposed to define, between the insert and the interior wall of ferrule, a passageway for the liquid to be transferred.

2. Device according to claim 1, characterized in that the insert (24) and the adapter form a unit.

3. Device according to claim 1, characterized in that the insert (66) is independent of the adapter.

4. Device according to claim 1, characterized in that the central cavity of the tubular ferrule (16, 58) comprises a truncated interior wall (21, 62) whose generatrices converge towards the transfer apparatus (10, 50).

5. Device according to claim 1, characterized in that the tubular ferrule (16, 58) comprises a truncated exterior wall (23, 64) whose generatrices diverge towards the transfer apparatus.

6. Device according to claim 5, characterized in that there is a 6% divergence in the truncated exterior wall (23, 64) of the tubular ferrule (16, 58).

7. Device according to claim 1, characterized in that the tip is a trocar (12), comprising a first truncated end portion (24), an intermediate portion (25) and a second conical end portion (26), in that said first end portion (24) constitutes the said insert and engages within the central cavity (20) of the tubular ferrule (16), in that said intermediate portion (25) has a larger exterior diameter than the central cavity, at its wide end, and in that the three portions of this trocar are traversed by a peripheral longitudinal groove (29) opening at least partially defining the channel for passage of the liquid to be transferred.

8. Device according to claim 6, characterized in that the intermediate portion (25) of the trocar is disposed to be in contact with the free end of the tubular ferrule (16).

9. Device according to claim 1, characterized in that the tip is an injection needle (60), in that the adapter is a bell-shaped cover (59) disposed to fit tightly over the tubular ferrule (58) and holding said injection needle, and in that the insert (66), independent of said cover, comprises a first truncated end portion (67) engaged within the central cavity of the tubular ferrule (58), an intermediate portion (68) and a second end portion (69) engaged within the interior cavity of the said bell-shaped cover.

10. Device according to claim 9, characterized in that the second end portion (69) of insert (66) is designed to occupy only a portion of the free space in the interior cavity of the bell-shaped cover (59), so as to form a transparent control chamber for viewing the passage of the liquid through the cover wall.

11. Device according to claim 9, characterized in that the intermediate portion (68) of the insert (66) is disposed to be in contact with the free end of the tubular ferrule (58) and with the interior wall of said bell-shaped cover.

12. Device according to claim 1, characterized in that the tip is a nasal spray applicator (90) comprising a rounded end portion (93) formed at the end of an insert (92) situated inside the tubular ferrule (91), and in that the passageway for the liquid to be transferred opens at the base of the rounded end portion (93) between the walls of the insert (92) and those of the tubular ferrule (91).

13. Device according to claim 12, characterized in that the passageway (94) comprises an end piece which opens at an angle of from 30 to 90 degrees and preferably at about 60 degrees in relation to the surface of the tubular ferrule and/or the insert in the area of the passageway opening.

14. Device according to claim 12, characterized in that the tip comprises a projected body (103) inside which there is mounted an insert (104) defining with the interior body wall a canal (105) of liquid, and in that said body comprises a stop flange (106) which constitutes a stop located at a predetermined distance from the opening of the canal (105) and disposed to determine the depth for applying the liquid inside the nostrils.

15. Device according to claim 1, characterized in that the tip is a dropper (110), and in that the insert (111) is provided with a flat element (113) at its upper extremity, said flat element comprising and opening (114) extending into the passageway for the liquid (115) disposed between the said insert and the said tubular ferrule (112).

16. Device according to claim 15, characterized in that the tip is surmounted by a cover (116) comprising an interior ring (117) disposed to ensure that the cover opens to allow the gas inside the distributor to flow out and to ensure close tightly.

17. Device according to claim 16, characterized in that the cover (116) comprises a base associated with an anti-bacterial agent.

Fig.IA

Fig.IB

Fig.IC

Fig.I

Fig.2A

Fig.2B

Fig.2C

Fig.2

Fig.4

Fig.3

Fig. 5

Fig. 6

*Fig. 7*

Fig. 8

*Fig. 9*